(19)

Europäisches
Patentamt
European
Patent Office
Office européen
des brevets

(11)    EP 4 768 437 A1

(12)    EUROPEAN PATENT APPLICATION

(43) Date of publication:
      **01.07.2026   Bulletin 2026/27**

(21) Application number: **25160454.2**

(22) Date of filing: **27.02.2025**

(51) International Patent Classification (IPC):
     *C01B 33/193* [(2006.01)]     *A61K 8/02* [(2006.01)]
     *A61K 8/25* [(2006.01)]     *A61Q 11/00* [(2006.01)]

(52) Cooperative Patent Classification (CPC):
     **C01B 33/193; A61K 8/0279; A61K 8/25;**
     **A61Q 11/00;** C01P 2004/61; C01P 2006/10;
     C01P 2006/12; C01P 2006/14; C01P 2006/16;
     C01P 2006/17; C01P 2006/90

(84) Designated Contracting States:
     **AL AT BE BG CH CY CZ DE DK EE ES FI FR GB**
     **GR HR HU IE IS IT LI LT LU LV MC ME MK MT NL**
     **NO PL PT RO RS SE SI SK SM TR**
     Designated Extension States:
     **BA**
     Designated Validation States:
     **GE KH MA MD TN**

(30) Priority:  **31.12.2024   CN 202411995220**

(71) Applicant: **Jin San Jiang (Zhaoqing) Silicon**
     **Material Co. Ltd**
     **Zhaoqing Guangdong 526238 (CN)**

(72) Inventors:
     • **Wang, Xianwei**
       **Zhaoqing, Guangdong, 526238 (CN)**

     • **Huang, Dan**
       **Zhaoqing, Guangdong, 526238 (CN)**
     • **He, Jinli**
       **Zhaoqing, Guangdong, 526238 (CN)**
     • **Cheng, Dehui**
       **Zhaoqing, Guangdong, 526238 (CN)**
     • **Mi, Shuang**
       **Zhaoqing, Guangdong, 526238 (CN)**
     • **Liu, Chenglin**
       **Zhaoqing, Guangdong, 526238 (CN)**
     • **Ren, Zhenxue**
       **Zhaoqing, Guangdong, 526238 (CN)**

(74) Representative: **Petraz, Gilberto Luigi et al**
     **GLP S.r.l.**
     **Viale Europa Unita, 171**
     **33100 Udine (IT)**

(54)    **ABRASIVE-TYPE SILICA FOR TOOTHPASTE WITH LOW SPECIFIC SURFACE AREA AND LOW PORE VOLUME AND PREPARATION METHOD THEREFOR**

(57)    The present disclosure belongs to the technical field of silica. The present disclosure particularly relates to an abrasive-type silica for toothpaste with a low specific surface area and a low pore volume and a preparation method therefor. The abrasive-type silica provided by the present disclosure has a specific surface area less than 2.79 $m^2/g$ and a pore volume less than 0.4 $cm^3/g$, has the advantages of high stability, better essence volatility, high dispersive performance, high cleanliness, good fluorine compatibility, and the like, is suitable for use in toothpaste, and complies with the requirements of European Union regulations. The preparation method for silica provided by the present disclosure has the advantages of a simple and stable process, easy condition control, low cost, and the like, and is easier for industrial production and popularization and application.

FIG. 1

EP 4 768 437 A1

**Description**

**TECHNICAL FIELD**

The present disclosure belongs to the technical field of silica. More particularly, the present disclosure relates to an abrasive-type silica for toothpaste with a low specific surface area and a low pore volume and a preparation method therefor.

**BACKGROUND**

Silica is an excellent toothpaste abrasive which has been developed rapidly in recent years, and has the advantages of good cleaning effect, strong polishing effect, good fluorine compatibility, good chemical stability, and the like, and is widely applied. Silica for toothpaste has a wide market space. The Chinese toothpaste industry also has a larger share in exports. The silica for toothpaste cannot contain a nanomaterial according to European Union policy regulations. As defined in the European Union regulations (Commission Recommendation 2011/696/EU of 18 October 2011 on the definition of nanomaterial (OJL 275, 20.10.2011, p. 38)), a solid particle is considered as a nanomaterial when 50% or more of particles of the solid particle based on the number size distribution satisfy that one or more external dimensions of these particles are within the size range of 1 nm to 100 nm. However, a material with a volume specific surface area less than 6 $m^2/cm^3$ needs not to be considered as a nanomaterial. The product particle diameter of the silica for toothpaste is characterized typically by using a weight median particle diameter D50. New small particles are continuously generated and grow during the formation of silica. The particles generated later have a small particle diameter. After being crushed, the silica product will generate a part of small particles having the particle diameter range of 1 nm to 100 nm. The number of these particles having a particle diameter less than 100 nm cannot be accurately characterized, and it cannot be accurately determined whether they are nanomaterials according to the definition of the European Union. The density of silica is 2.15 $g/cm^3$, so the requirement of "a volume specific surface area less than 6 $m^2/cm^3$" in the European Union regulations can be converted into "a specific surface area of silica less than 2.79 $m^2/g$". Therefore, to ensure that the generated silica can satisfy the requirements of the European Union regulations, it is necessary to develop silica with a specific surface area less than 2.79 $m^2/g$ to satisfy the requirement of a volume specific surface area less than 6 $m^2/cm^3$ in the European Union regulations.

The specific surface area (BET) of silica is 1000 times the ratio of 4 times the pore volume to the pore diameter. Therefore, decreasing the specific surface area can be achieved by decreasing the pore volume or increasing the pore diameter. However, at present, the existing pore volume of silica is substantially above 0.4 $cm^3/g$, and there are few reports concerning the low pore volume. The pore volume is mostly involved in the production of silica used in other fields rather than toothpaste, which mostly pursues the large pore volume to improve the adsorption performance thereof. However, for the silica for toothpaste, it is better to select an approach for decreasing the pore volume. The use for toothpaste does not require the adsorption performance of the silica and the reaction activity thereof, but requires the silica to keep inert so that the silica serves as an abrasive quietly, and the compatibility between the silica and other components in toothpaste is higher. The pore volume of silica is small, then the internal pores are relatively fewer, the available active sites are fewer, and the capacity to adsorb and load other substances is weaker. If the capacity to adsorb an essence is weak, then the volatility of the essence is better so that the cost of toothpaste can be reduced (the essence is the most expensive raw material in the toothpaste formula). Moreover, the silica with a low pore volume is more dense, and the corresponding dispersive performance and cleaning performance will be better. Therefore, for the silica for toothpaste, decreasing the pore volume as much as possible while pursuing a low specific surface area is more advantageous. However, for the silica with a low specific surface area, to prepare silica with a pore volume less than 0.4 $cm^3/g$, finer control of pore structure and pore size is required, which has a higher difficulty and is difficult to achieve a low specific surface area and a low pore volume simultaneously. Furthermore, due to the inherent microstructure characteristic of silica, it originally forms a relatively large number of pores, and it is difficult to decrease the pore volume. The prior art lacks a means for precisely controlling pore formation. Therefore, it is common in the existing solution to decrease the specific surface area of silica by increasing the pore diameter.

The existing specific surface area of silica is also generally higher. The silica with a low specific surface area prepared in patent CN201810273705 has a specific surface area within the range of 30.3-38.7 $m^2/g$, and the specific surface area still cannot satisfy the non-nano requirements of the European Union. The silica prepared in patent US12145852B2 has a specific surface area within the range of 0.1-3.4 $m^2/g$, but the pore volume and pore diameter data is not given. The silica prepared in the patent is mainly used for semiconductor encapsulation materials.

Therefore, the exploration and development of abrasive-type silica for toothpaste with a specific surface area less than 2.79 $m^2/g$ and a pore volume less than 0.4 $cm^3/g$ has important value for the export of raw materials of silica for toothpaste and toothpaste industry in China.

## SUMMARY

The present disclosure aims to develop an abrasive-type silica for toothpaste with a specific surface area less than 2.79 $m^2$/g and a pore volume less than 0.4 $cm^3$/g.

A purpose of the present disclosure is to provide an abrasive-type silica for toothpaste with a low specific surface area and a low pore volume.

Another purpose of the present disclosure is to provide a preparation method for the above abrasive-type silica for toothpaste with a low specific surface area and a low pore volume.

The above purposes of the present disclosure are implemented through the following technical solutions: The present disclosure provides an abrasive-type silica for toothpaste with a low specific surface area and a low pore volume, satisfying the following indexes:

(1) a specific surface area is: 0.01-2.79 $m^2$/g;
(2) a pore volume is: 0.00004-0.3 $cm^3$/g;
(3) a pore diameter is: 5-26 nm; and
(4) a median particle diameter D50 is: 1-25 $\mu$m.

Further, the abrasive-type silica for toothpaste with a low specific surface area and a low pore volume has the following performance indexes:

(5) a fluorine compatibility is: 75%-99%;
(6) a copper sheet wear value is: 6-25 mg per 10,000 revolutions;
(7) an RDA value is: 180-360;
(8) a PCR value is: 80-160;
(9) an apparent density is: 0.02-1.5 g/ml; and
(10) an oil absorption value is: 10-125 g/100g.

Preferably, the abrasive-type silica for toothpaste with a low specific surface area and a low pore volume satisfies the following indexes:

(1) the specific surface area is: 0.01-2.79 $m^2$/g;
(2) the pore volume is: 0.00004-0.1 $cm^3$/g;
(3) the pore diameter is: 15-26 nm; and
(4) the median particle diameter D50 is: 1-20 $\mu$m.

Further, the abrasive-type silica for toothpaste with a low specific surface area and a low pore volume has the following performance indexes:

(5) the fluorine compatibility is: 80%-99%;
(6) the copper sheet wear value is: 10-20 mg per 10,000 revolutions;
(7) the RDA value is: 180-260;
(8) the PCR value is: 100-160;
(9) the apparent density is: 0.3-1.5 g/ml; and
(10) the oil absorption value is: 10-60 g/100g.

More preferably, the abrasive-type silica for toothpaste with a low specific surface area and a low pore volume satisfies the following indexes:

(1) the specific surface area is: 0.1-2.5 $m^2$/g;
(2) the pore volume is: 0.00004-0.02 $cm^3$/g;
(3) the pore diameter is: 15-26 nm; and
(4) the median particle diameter D50 is: 1-16 $\mu$m.

Further, the abrasive-type silica for toothpaste with a low specific surface area and a low pore volume has the following performance indexes:

(5) the fluorine compatibility is: 90%-99%;
(6) the copper sheet wear value is: 10-20 mg per 10,000 revolutions;

(7) the RDA value is: 200-250;

(8) the PCR value is: 100-160;

(9) the apparent density is: 0.5-1.0 g/ml; and

(10) the oil absorption value is: 10-60 g/100g.

The abrasive-type silica provided by the present disclosure has a specific surface area less than 2.79 $m^2$/g and a pore volume less than 0.4 $cm^3$/g, has the advantages of high stability, better essence volatility, high dispersive performance, high cleanliness, good fluorine compatibility, and the like, is suitable for use in toothpaste, complies with the requirements of European Union regulations, and is suitable for being applied to toothpastes intended for export.

In a specific embodiment, the essence volatility of the abrasive-type silica provided by the present disclosure can be measured by using GC-MS or weight-method test, an essence is added into the abrasive-type silica provided by the present disclosure, and then test is carried out; in addition, to facilitate simulation of an influence of a toothpaste environment on adsorption of silica to essence, the essence may also be added into the toothpaste comprising the abrasive-type silica of the present disclosure for test.

As a first embodiment, specifically, GC-MS is used for test, and the essence volatility of the abrasive-type silica of the present disclosure is ≥90%; and a test method for the essence volatility includes: adding an essence into the abrasive-type silica of the present disclosure or a toothpaste including the abrasive-type silica of the present disclosure, and using the GC-MS for test to measure a proportion of the essence in a headspace gas, i.e., the essence volatility.

Preferably, the essence volatility of the abrasive-type silica of the present disclosure is 90%-99.99%.

More specifically, the essence volatility may be 91%, 92%, 93%, 94%, 95%, 96%, 97%, 98%, 99%, 99.9%, and the like, or within an interval range formed by any of the above values, e.g., 96.275%-98.586%, 98.069%-98.586%, etc., and the present disclosure is not limited thereto.

More preferably, the essence volatility of the abrasive-type silica of the present disclosure is 90.906%-98.586%.

As a more specific embodiment, a method for testing the essence volatility of the abrasive-type silica of the present disclosure by using GC-MS is operated as follows: components are mixed well and sealed for storage for 5-10 days, and then GC-MS test is carried out. More specifically, a temperature for the storage is 25°C-40°C.

Referably, test conditions of GC-MS are as follows: chromatographic conditions: heating-up program: initial temperature of 50°C, rising to 300°C at 5 °C/min (keeping for 20 minutes); carrier gas: helium (99.999%); carrier gas flow rate: 1.0 mL/min; injection port temperature: 260°C; and split ratio: 10:1.

Mass spectrometry conditions: transmission line temperature: 280°C; ionization mode: Electron Ionization (EI); ionization energy: 70 eV; ion source temperature: 230°C; quadrupole rod temperature: 150°C; solvent delay time: 3 minutes; and full scan monitoring mode, with a scan range of 30-500 amu.

A GC-MS detection instrument is, for example, an Agilent gas chromatograph-mass spectrometer (instrument model: 8890/5977B GC/MSD).

As a second embodiment, the essence volatility of the abrasive-type silica provided by the present disclosure may also be tested by using a weight method. Specifically, the weight method is used for test, and the essence volatility of the abrasive-type silica of the present disclosure is as follows: when the weight method is used for test, the essence volatility for 14 days is ≥40%; and a test method for the essence volatility includes: adding the essence into the abrasive-type silica of the present disclosure or the toothpaste including the abrasive-type silica of the present disclosure, and testing a change in total weight after 14 days, wherein the change in total weight tested is the essence volatility.

Specifically, the "change in total weight" means a reduction in a total weight of the essence and the silica or a total weight of the essence and the toothpaste after 14 days compared with before 14 days.

Preferably, the essence volatility of the abrasive-type silica of the present disclosure is as follows: when the weight method is used for test, the essence volatility for 14 days is 40%-90%. More preferably, the essence volatility for 14 days is 46%-78%. More preferably, the essence volatility for 14 days is 60%-78%.

More specifically, the essence volatility for 14 days may be 45%, 50%, 55%, 60%, 65%, 70%, 75%, 80%, 85%, and the like, or within an interval range formed by any of the above values, e.g., 70%-78%, 65%-80%, etc., and the present disclosure is not limited thereto.

In the above methods for testing the essence volatility, if an essence is added into the toothpaste including the abrasive-type silica of the present disclosure for test, the toothpaste includes, but is not limited to, one or more of a thickener, a sweetening agent, an antibacterial agent, an abrasive, a wetting agent, a pigment or coloring agent, a flavoring agent, a foaming agent, an essence, and a solvent. Preferably, the wetting agent includes one or more of glycerol, sorbitol, xylitol, and propylene glycol. Preferably, the foaming agent is selected from one or more of sodium lauryl sulfate, cocamidopropyl betaine, alkyl glycoside, alkyl sulfonate, and alkylbenzene sulfonate. Preferably, the solvent is an alcoholic solvent and/or water.

In addition, the type of the essence may be an essence conventionally included in the toothpaste in the art. Preferably, the essence is selected from one or more of menthol, menthone, limonene, methyl salicylate, carvone, and anethole.

Specifically, the toothpaste includes: 1-1.5 parts by weight of a wetting agent, 1-1.5 parts by weight of silica, 0.1-0.5 parts by weight of a foaming agent, 0.05-0.3 parts by weight of an essence, and 1-1.5 parts by weight of a solvent.

In addition, the present disclosure further provides use of the abrasive-type silica for toothpaste with a low specific surface area and a low pore volume in preparation of an oral abrasive, a cleaning aid, or toothpaste.

Further, the present disclosure further provides an oral composition, including the above abrasive-type silica for toothpaste with a low specific surface area and a low pore volume, and an oral acceptable carrier.

Optionally, in the oral composition, a content of the abrasive-type silica is 0.5%-90.0% by mass.

Preferably, in the oral composition, the content of the abrasive-type silica is 1.0%-80.0% by mass.

In the oral composition, the abrasive-type silica is present as a sole abrasive in the oral composition.

In the oral composition, the abrasive-type silica is present as an abrasive and/or a cleaning aid.

As an optional embodiment, the oral composition is toothpaste.

The present disclosure also explores and optimizes the preparation method for the above abrasive-type silica for toothpaste with a low specific surface area and a low pore volume, and develops a method for preparing the abrasive-type silica for toothpaste with a low specific surface area and a low pore volume based on a precipitation method. The precipitation method is simpler and more stable in process, easy in condition control, and easier for industrial production.

Specifically, the present disclosure provides a method for preparing the above abrasive-type silica for toothpaste with a low specific surface area and a low pore volume, including the following steps:

1) adding a sodium sulfate solution into a reaction vessel (e.g., a reaction kettle);

2) adding a sodium silicate solution and stirring well until a pH of a reaction mixture is 7-14;

3) heating up until a temperature of the reaction mixture is 60°C-97°C; and maintaining a reaction temperature, stirring at a rotation speed of 200-600 revolutions per minute, slowly adding the sodium silicate solution and a sulfuric acid solution simultaneously, with a flow rate of the sodium silicate solution being 5-30 m$^3$/h, and maintaining a pH within a range of 7-10 by adjusting a flow rate of the sulfuric acid solution;

4) continuously adding the sodium silicate solution until a volume ratio of the sodium silicate solution to the sodium sulfate solution in step 1) reaches (1.5-2):1 (at this time, reaction time is about 10,000-30,000 seconds); and continuously adding the sulfuric acid solution until the pH of reaction mixture reaches 4-7, and stopping addition of the sulfuric acid solution;

5) continuing to stir, carrying out heat preservation for aging for 10-60 minutes to prepare a silica slurry; and

6) subjecting the silica slurry to filtering, washing, drying, and crushing to obtain a finished product,

wherein the sodium sulfate solution has a mass percent concentration of 6%-20%;
the sodium silicate solution has a concentration of 1-2.5 mol/L; and
the sulfuric acid solution has a concentration of 1-2 mol/L.

Preferably, the sodium sulfate solution has the mass percent concentration of 8%-20%.

Preferably, the sodium silicate solution has the concentration of 1.5-2.5 mol/L.

Preferably, the pH of reaction mixture in step 2) is 7-12, respectively.

Preferably, the flow rate of the sodium silicate solution in step 3) is 5-7 m$^3$/h.

Preferably, it is recommended to adjust the flow rate of the sulfuric acid solution in step 3) within a range of 10-12 m$^3$/h.

Preferably, a stirring speed in step 3) is 200-400 revolutions per minute.

Specifically, the present disclosure provides another method for preparing the above abrasive-type silica for toothpaste with a low specific surface area and a low pore volume, including the following steps:

1) adding a sodium sulfate solution and a sodium silicate solution into a reaction vessel (e.g., a reaction kettle), and heating up to 90°C-95°C;

2) stirring at a rotation speed of 200-600 revolutions per minute, adding a sulfuric acid solution into the reaction vessel (e.g., the reaction kettle), and adjusting a process pH to 7.0-7.5;

3) slowly adding the sodium silicate solution and the sulfuric acid solution simultaneously (simultaneously dropwise adding acid and base), with an addition speed of the sodium silicate solution being 5-20 m$^3$/h; and maintaining a pH of a solution at 7.0-7.5 by adjusting a flow rate of the sulfuric acid solution, wherein silica particles growing under the condition of the process pH being partial neutral have poorer activity so that collision between particles can be reduced, the acid and base neutralization rate in a reaction system is higher, crystal grains grow rapidly, and since the end of a sol bond has high activity and lower aggregation density, active groups are easy to be close to each other, thereby promoting the growth of the bond, forming a linear chain structure which facilitates the formation of dense particles, and reduces the specific surface area of the particles;

4) continuously adding the sodium silicate solution until a volume ratio of the sodium silicate solution to the sodium sulfate solution in step 1) reaches (3-8):1; and continuously adding the sulfuric acid solution until a pH of a reaction mixture is 4-7, and stopping addition of the sulfuric acid solution;

5) continuing to stir, carrying out heat preservation for aging for 30 minutes to prepare a silica slurry; and

6) filtering the silica slurry with a diaphragm, and recovering the sodium sulfate solution; and then washing, spray-drying, and jet mill crushing to prepare silica with a low specific surface area and a low pore volume,

> wherein the sodium sulfate solution has a mass percent concentration of 6%-20%;
> the sodium silicate solution has a concentration of 1-2.5 mol/L; and
> the sulfuric acid solution has a concentration of 1-2 mol/L.

Preferably, in step 1), a volume ratio of the sodium sulfate solution to the sodium silicate solution is 1:(0.05-0.2).

More preferably, the volume ratio of the sodium sulfate solution to the sodium silicate solution in step 1) is 1:0.1.

Preferably, the flow rate of the sulfuric acid solution in step 2) is 5-10 $m^3$/h.

More preferably, the flow rate of the sulfuric acid solution in step 2) is 5 $m^3$/h.

Preferably, in step 4), the addition of the sodium silicate solution is stopped until the volume ratio of the sodium silicate solution to the sodium sulfate solution in step 1) reaches 5:1.

Preferably, it is recommended to adjust the flow rate of the sulfuric acid solution in step 3) within a range of 10-12 $m^3$/h.

In the preparation method of the present disclosure, sodium silicate, sulfuric acid and sodium sulfate as reaction raw materials react to prepare the abrasive-type silica with a low specific surface area and a low pore volume. In the preparation method of the present disclosure, the precipitation method is used for reaction, the sodium sulphate solution with a high concentration is added as a reaction aid at the beginning of the reaction, the reaction system is weakly basic by dropwise adding the sodium silicate solution, and the reaction is carried out at 60°C-97°C, so that the silica initial particles have a denser structure, thereby decreasing the specific surface area and the low pore volume of the silica; then, the addition means of simultaneously dropwise adding acid and base is used to keep the pH value in the reaction process to be weakly basic so as to avoid the generation of a gel, thereby ensuring the low specific surface area and low pore volume of the finished product. At the end of the reaction, an acid is added for titration until the terminal pH value is 4.0-7.0, and aging is carried out for 10-60 minutes in the acidic system under the condition of heat preservation, so as to stabilize the structure of the silica.

The abrasive-type silica prepared according to the above method has an extremely low specific surface area and an extremely low pore volume. Specifically, the specific surface area is 0.01-2.79 $m^2$/g, and the pore volume is 0.0006-0.3 $cm^3$/g. Moreover, the abrasive-type silica has the fluorine compatibility of 90%-96%. The abrasive-type silica has the advantages of high stability, better essence volatility, high dispersive performance, high cleanliness, good fluorine compatibility, and the like, is suitable for use in toothpaste, and complies with the requirements of European Union regulations. Moreover, the method is simple, stable, and low in cost.

In addition, the present disclosure also gropes for the calcination process, and the calcination can also form silica with a relatively low pore volume and a relatively low specific surface area. However, compared with the improved preparation method provided above by the present disclosure, although the calcination can also form the relatively low pore volume and the relatively low specific surface area, the requirements for the calcination temperature and time are relatively strict, and if they are not controlled well, excessive pores are easily generated, so that the silica satisfying the requirements of a low specific surface area and a low pore volume can be prepared only by strictly controlling the conditions. Furthermore, the calcination means is prone to nonuniformity and instability, and has a higher cost and energy consumption. Therefore, industrialization of the calcination process is inferior to the improved preparation process based on a precipitation method provided above by the present disclosure.

The present disclosure has the following beneficial effects:

The abrasive-type silica of the present disclosure has the following advantages:

> (1) A low specific surface area: a BET specific surface area is less than 2.79 $m^2$/g. The toothpaste prepared from the silica with a low specific surface area has higher stability. Moreover, the silica with a low specific surface area less than 2.79 $m^2$/g can satisfy the definition of the non-nanomaterial of the European Union, and belongs to non-nanomaterials. The silica has a broader prospect than the existing abrasive-type silica.
> (2) A low pore volume: the silica has the advantages of weak capacity to adsorb an essence, better essence volatility, and reducing the cost of toothpaste. Moreover, the silica with a low pore volume is more dense, and the corresponding dispersive performance and cleaning performance will be better.
> (3) A good fluorine compatibility: the fluorine compatibility is within a range of 75%-99%.
> (4) The preparation method for the abrasive-type silica for toothpaste with a low specific surface area and a low pore volume provided by the present disclosure is simple and stable in process, easy in condition control, low in cost, and easier for industrial production and popularization and application.

## BRIEF DESCRIPTION OF DRAWINGS

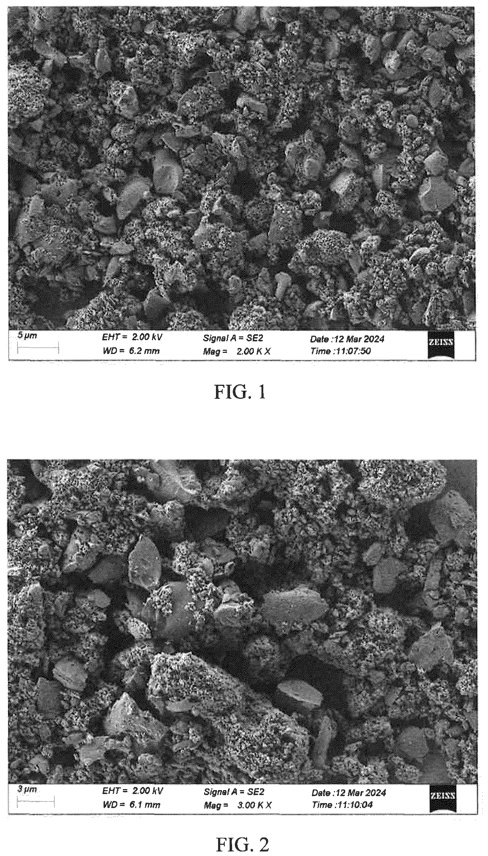

FIG. 1 is a scanning electron micrograph of silica of Example 1.
FIG. 2 is a scanning electron micrograph of silica of Example 5.

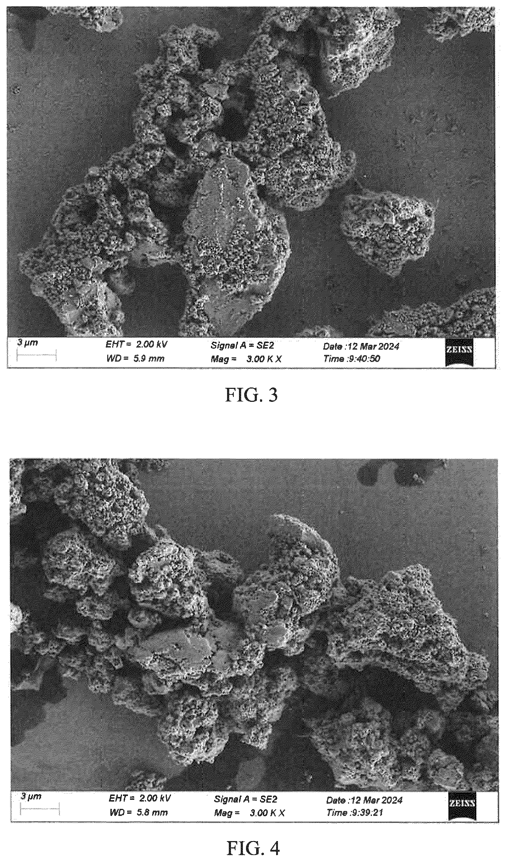

FIG. 3 is a scanning electron micrograph of silica of Comparative Example 4.
FIG. 4 is a scanning electron micrograph of silica of Comparative Example 7.

## DETAILED DESCRIPTION OF EMBODIMENTS

The present disclosure is further illustrated below in combination with the drawings of the specification and specific examples, but the examples do not limit the present disclosure in any form.

The reagents, methods, and devices employed in the present disclosure are those conventional in the technical field, unless otherwise specified.

The reagents and materials used in the following examples are all commercially available, unless otherwise specified.

A test method for testing silica particle indexes of the present disclosure was as follows:

1. Method for measuring an oil absorption value of powder:
For the method for testing the oil absorption value of powder, reference was made to ASTM-D281.
2. Method for measuring a BET specific surface area of powder:
According to instructions for use, the specific surface area, the pore volume, and the pore diameter were tested by using a JW-BK112 type static nitrogen adsorption apparatus.
3. Method for measuring a fluorine compatibility of powder

(1) 7.00 g of abrasive-type silica or 2.00 g of thickening-type silica (generally weighing 2 parts for parallel test) were weighed into a plastic bottle, and 30.00 g of NaF (1,624 ppm stock solution) was slowly added. Sealing (preventing the liquid from volatilizing) was carried out to completely wet $SiO_2$, and vibration was carried out. The mixture was placed on a rotating frame at 60°C for rotary heating for 1 hour, cooled, and centrifuged in a centrifugal machine at 15,000 rpm for 15 minutes. The supernatant was taken.
(Note: without the rotating frame, placing the sample bottle in a temperature-constant oven at 60°C for heating, and shaking frequently by hand. Using a #4 rotor and 5 ml centrifuge tubes, one sample was divided into 2 centrifuge tubes, and thus 2 samples were put into 4 centrifuge tubes in total)
(2) 2.00 g of the supernatant was weighed precisely, and then a 9 times (18 g) EDTA/THAM buffer was weighed into a plastic bottle for testing.
(3) A reference electrode and an F ion electrode were mounted, adjusted to mv in pH/mv, and washed with distilled water to above 370 mv (washed in advance) at a room temperature.
(4) The washed glass electrodes were wiped dry. A low standard liquid (10%) was firstly measured. The number was recorded after being stable. The glass electrodes were washed with distilled water. Then, a high standard liquid (90%) was measured. The number was recorded. After the standard liquids were measured, the glass electrodes were washed and wiped dry. A sample to be tested was tested. Stable reading was carried out. Data was recorded.
(5) Calculation

$$y1 = ax1 + b$$

$$y2 = ax2 + b$$

y1 was a high standard liquid concentration of 90%, y2 was a low standard liquid concentration of 10%, x1 was a high standard liquid reading, and x2 was a low standard liquid reading. The values of a and b were calculated.
y = ax + b, where x was a reading of a sample to be tested, and y was a fluorine compatibility of a sample to be tested.

4. PCR test method

For the pellicle cleaning ratio (PCR), reference was made to GB/T 43576-2023 *"Oral care and cleaning products-Laboratory method of effect removal of extrinsic stain for toothpastes"*.

5. RDA test method

The radioactive dentin abrasion (RDA) value was determined by Annex B, International Organization for Standardization (ISO) 11609:2010 (E). The test was repeated at least three times, and the average value was calculated to obtain the average RDA.

6. For the test method for copper loss, reference to JSJ-C-109 overhard particle test

First, it was confirmed that the overhard particle tester could be powered on, and then the power source was turned off to continue the next operation.

Two copper sheets were taken, washed clean with distilled water, blown dry with a blow dryer, placed in a desiccator for 15 minutes, and then taken out by wearing gloves. The weights m1 and m2 (in a unit of mg) of the two copper sheets before wear were weighed respectively. After weighing, the two copper sheets were placed in a tank of an overhard particle measuring instrument and fixed.

20.0 g of a silica or abrasive particle sample was accurately weighed and evenly dispersed in a 120.0 g sorbitol solution, and the obtained slurry was transferred to the tank of the overhard particle measuring instrument.

The overhard particle measuring instrument was turned on, and the copper sheets were continuously abrased 10,000 times by an abrasion head in the test slurry.

After the abrasion was completed, the power source was turned off first, then the copper sheets were taken out, washed clean with tap water, washed twice with distilled water, finally blown dry with a blow dryer, placed in a desiccator for 15 minutes, and then taken out by wearing gloves. The weights m1 and m2 (in a unit of mg) of the two copper sheets after wear were weighed respectively.

The difference between the weights of the two copper sheets before and after wear, i.e., the wear values $\Delta m_1$ and $\Delta m_2$ of copper loss (in a unit of mg, the average value thereof was taken as a final result. The deviation of the parallel test result was not more than 20%).

7. A test method for silica essence volatility of the present disclosure was as follows:

(1) Gas chromatography-mass spectrometry (GC-MS) test method:

A 20 ml headspace vial was taken. 1.25 g of glycerol, 1.25 g of water, 1.25 g of silica, 0.25 g of a 29% sodium lauryl sulfate solution (SLS solution), and 0.125 g of an essence for toothpaste (source: Guangzhou Tufu Perfume Technology Co., Ltd., dimenthol essence TF71438 (liquid)) were added. The caps were put on to ensure sealing. Oscillatory mixing was carried out for 10 minutes. Storage was carried out at 40°C for one week. The essence components in a headspace gas and the relative contents corresponding to the essence components were measured by using an Agilent gas chromatograph-mass spectrometer (instrument model: 8890/5977B GC/MSD).

Test parameters for GC-MS were as follows:

Chromatographic conditions: heating-up program: initial temperature of 50°C, rising to 300°C at 5 °C/min (keeping for 20 minutes); carrier gas: helium (99.999%); carrier gas flow rate: 1.0 mL/min; injection port temperature: 260°C; and split ratio: 10:1;

Mass spectrometry conditions: transmission line temperature: 280°C; ionization mode: Electron Ionization (EI); ionization energy: 70 eV; ion source temperature: 230°C; quadrupole rod temperature: 150°C; solvent delay time: 3 minutes; and full scan monitoring mode, with a scan range of 30-500 amu.

(2) Weight-method test method:

Materials and reagents: 250 ml plastic bottles, a one ten-thousandth balance, silica, and a peppermint essence.

Preparation of samples:

A test group: 5 g of silica and 2 g of an essence;
A blank group: 5 g of silica;
Three samples were prepared for each group for parallel test.
Test: all samples were placed at room temperature, the weights were recorded daily, and the test lasted for 14 days, and the weight loss of each sample was recorded for data processing and analysis.

**Example 1 Preparation of Silica by Precipitation Method**

(I) Raw materials:
Sodium sulfate solution: a sodium sulfate solution with a mass percent concentration of 12.0% was prepared.

Sodium silicate solution: solid sodium silicate with a modulus of 3.3-3.45 was used and liquefied at a high temperature, and then water was added to prepare a sodium silicate solution with a concentration of 2.0 mol/L.

Sulfuric acid solution: a sulfuric acid solution with a concentration of 1.3 mol/L was prepared.

(II) Preparation of silica, having the following steps:

1) 17.5 $m^3$ of the sodium sulfate solution was added into a reaction vessel;

2) the sodium silicate solution was added, and the pH of reaction mixture was regulated to 8.5;

3) heating up was carried out until the temperature of reaction mixture was 80°C, the reaction temperature was maintained, stirring was carried out at a rotation speed of 400 revolutions per minute, and the sodium silicate solution and the sulfuric acid solution were slowly added simultaneously (acid and base were dropwise added simultaneously), with an addition speed (flow rate) of the sodium silicate solution being 5 $m^3$/h; and the pH of the solution was maintained at 8.5 by adjusting the flow rate of the sulfuric acid solution;

4) the addition of the sodium silicate solution was stopped when the sodium silicate solution was added to 35 $m^3$ (at this time, the reaction time was about 25,200 seconds); and the sulfuric acid solution was added continuously until the pH of reaction mixture was 4.5, and the addition of the sulfuric acid solution was stopped;

5) stirring continued, heat preservation for aging was carried out for 30 minutes to prepare a silica slurry; and

6) the silica slurry was filtered with a diaphragm, and the sodium sulfate solution was recovered; and then washing, spray-drying, and jet mill crushing were carried out to prepare an abrasive-type silica with a low specific surface area and a low pore volume.

**Example 2 Preparation of Silica by Precipitation Method**

Compared with Example 1, this example only differs in that the sodium sulfate solution had a mass percent concentration of 17%.

**Example 3 Preparation of Silica by Precipitation Method**

Compared with Example 1, this example only differs in that the sodium silicate solution had a concentration of 1.3 mol/L.

**Example 4 Preparation of Silica by Precipitation Method**

Compared with Example 1, this example only differs in that the pH of reaction mixture in step 2) to step 3) was maintained at 7.5.

**Example 5 Preparation of Silica by Precipitation Method**

Compared with Example 1, this example only differs in that the reaction temperature in step 3) was maintained at 95°C.

**Example 6 Preparation of Silica by Precipitation Method**

Compared with Example 1, this example only differs in that the stirring speed in step 3) was 600 revolutions per minute.

**Example 7 Preparation of Silica by Precipitation Method**

Compared with Example 1, this example only differs in that the sulfuric acid solution had a concentration of 1.8 mol/L.

**Example 8 Preparation of Silica by Precipitation Method**

Compared with Example 1, this example only differs in that the flow rate of the sodium silicate solution in step 3) was 7 m$^3$/h. The sulfuric acid solution still had a concentration of 1.3 mol/L, and the pH was maintained at 8.5 by adjusting the flow rate of the sulfuric acid solution, with the flow rate of base remaining constant.

**Example 9 Preparation of Silica by Precipitation Method**

Compared with Example 1, this example only differs in that the addition amount of the sodium silicate solution in step 4) was 28 m$^3$.

**Example 10 Preparation of Silica by Precipitation Method**

Compared with Example 1, this example only differs in that the time for the heat preservation for aging in step 5) was 50 minutes.

**Comparative Example 1**

Compared with Example 1, this comparative example only differs in that the sodium sulfate solution had a mass percent concentration of 5%.

**Comparative Example 2**

Compared with Example 1, this comparative example only differs in that the sodium silicate solution had a concentration of 0.8 mol/L.

**Comparative Example 3**

Compared with Example 1, this comparative example only differs in that the pH of reaction mixture in step 2) to step 3) was maintained at 6.5.

**Comparative Example 4**

Compared with Example 1, this comparative example only differs in that the stirring speed in step 3) was 800 revolutions per minute.

**Comparative Example 5**

Compared with Example 1, this comparative example only differs in that the reaction temperature in step 3) was maintained at 50°C.

**Comparative Example 6**

Compared with Example 1, this comparative example only differs in that the sulfuric acid solution had a concentration of 3 mol/L.

**Comparative Example 7**

Compared with Example 1, this comparative example only differs in that the flow rate of the sodium silicate solution in step 3) was 35 m$^3$/h. The sulfuric acid solution still had a concentration of 1.3 mol/L, and the pH was maintained at 8.5 by adjusting the flow rate of the sulfuric acid solution, with the flow rate of base remaining constant.

**Comparative Example 8**

Compared with Example 1, this comparative example only differs in that the addition amount of the sodium silicate solution in step 4) was 40 m$^3$.

**Comparative Example 9**

Compared with Example 1, this comparative example only differs in that the terminal pH of the reaction mixture in step 4) was 3.0.

**Comparative Example 10**

Compared with Example 1, this comparative example only differs in that the time for the heat preservation for aging in step 5) was 2 minutes.

**Performance Test for Silica Prepared in Examples 1-10 and Comparative Examples 1-10**

The performance of silica in Examples 1-10 and Comparative Examples 1-10 was tested. The test results are shown in Table 1 below:

The silica in Examples 1-10 of the present disclosure had a fluorine compatibility of 90%-96% and a specific surface area of <2.79 $m^2/g$, which could satisfy the requirements of the European Union for non-nanomaterials, and the silica had a pore volume of 0.0006-0.02 $cm^3/g$ and had excellent essence volatility. Moreover, the dispersive performance and cleaning performance were excellent.

However, in Comparative Examples 1-10, the specific surface area was >2.79 $m^2/g$, which could not satisfy the requirements of the European Union for non-nanomaterials; the pore volume was significantly higher than that in all the examples; the fluorine compatibility was also significantly lower than that in all the examples.

Table 1 Results of Performance Test for Silica in Examples 1-10 and Comparative Examples 1-10

| Item | Fluorine compatibility of powder (%) | BET ($m^2/g$) | Pore volume $cm^3/g$ | Pore diameter nm | RDA | PCR | D50 $\mu m$ | Apparent density g/ml | Oil absorption value g/100g | Copper loss mg |
|---|---|---|---|---|---|---|---|---|---|---|
| Example 1 | 95 | 1.1428 | 0.0067 | 23.4464 | 360.3 | 153.8 | 24.58 | 0.98 | 14 | 20.0 |
| Example 2 | 90 | 2.4943 | 0.0105 | 16.9320 | 253.2 | 122.6 | 10.33 | 0.87 | 31 | 17.8 |
| Example 3 | 93 | 0.1861 | 0.0010 | 21.0498 | 330.1 | 135.2 | 8.86 | 1.18 | 24 | 18.9 |
| Example 4 | 91 | 2.4393 | 0.0142 | 23.4325 | 221.4 | 118.1 | 15.24 | 0.89 | 28 | 16.6 |
| Example 5 | 92 | 0.1224 | 0.0007 | 22.8815 | 308.6 | 132.3 | 8.24 | 0.88 | 32 | 18.5 |
| Example 6 | 90 | 1.4531 | 0.0084 | 23.1214 | 254.1 | 121.2 | 12.19 | 0.73 | 48 | 15.3 |
| Example 7 | 93 | 2.2072 | 0.0104 | 18.8462 | 225.2 | 117.6 | 13.79 | 0.84 | 39 | 17.2 |
| Example 8 | 93 | 1.3064 | 0.0065 | 18.8835 | 216.6 | 115.6 | 14.48 | 0.91 | 29 | 16.3 |
| Example 9 | 95 | 1.5932 | 0.0094 | 23.6003 | 211.9 | 109.6 | 7.74 | 0.78 | 42 | 16.7 |
| Example 10 | 96 | 0.0924 | 0.0006 | 25.9516 | 303.3 | 145.6 | 5.54 | 1.43 | 22 | 19.8 |
| Comparative Example 1 | 84 | 23.262 1 | 0.1101 | 18.9321 | 182.4 | 101.9 | 12.12 | 0.66 | 69 | 14.3 |
| Comparative Example 2 | 88 | 4.1454 | 0.0192 | 17.2412 | 211.3 | 110.2 | 19.6 | 0.78 | 58 | 18.8 |
| Comparative Example 3 | 87 | 38.442 5 | 0.2033 | 21.1536 | 187.5 | 100.8 | 10.68 | 0.67 | 87 | 12.4 |
| Comparative Example 4 | 89 | 4.4993 | 0.0125 | 10.1216 | 311.6 | 125.9 | 14.6 | 0.86 | 54 | 18.9 |
| Comparative Example 5 | 88 | 48.794 2 | 0.2023 | 16.5839 | 188.3 | 100.5 | 17.8 | 0.72 | 89 | 13.6 |
| Comparative Example 6 | 83 | 6.4801 | 0.0225 | 13.8886 | 267.2 | 127.5 | 6.4 | 0.88 | 49 | 17.9 |

(continued)

| Item | Fluorine compatibility of powder (%) | BET (m²/g) | Pore volume cm³/g | Pore diameter nm | RDA | PCR | D50 μm | Apparent density g/ml | Oil absorption value g/100g | Copper loss mg |
|---|---|---|---|---|---|---|---|---|---|---|
| Comparative Example 7 | 86 | 15.132 2 | 0.0941 | 24.8819 | 249.6 | 120.8 | 12.60 | 0.71 | 52 | 17.2 |
| Comparative Example 8 | 89 | 7.7368 | 0.0362 | 18.7413 | 286.9 | 129.4 | 4.46 | 0.94 | 92 | 19.1 |
| Comparative Example 9 | 80 | 9.1341 | 0.0323 | 14.1446 | 317.3 | 134.2 | 12.28 | 0.96 | 56 | 19.4 |
| Comparative Example 10 | 84 | 11.774 9 | 0.0431 | 14.6412 | 323.8 | 135.7 | 10.59 | 0.89 | 44 | 19.8 |

Table 2 Results of GC-MS Test in Examples 1-10 and Comparative Examples 1-10

| Item | Menthol (%) | Menthone (%) | Limonene (%) | Methyl salicylate (%) | Carvone (%) | Anethole (%) | Sum (%) |
|---|---|---|---|---|---|---|---|
| Example 1 | 65.037 | 9.740 | 3.491 | 9.378 | 5.436 | 3.478 | 96.560 |
| Example 2 | 52.146 | 8.142 | 7.181 | 9.962 | 7.136 | 6.339 | 90.906 |
| Example 3 | 63.158 | 9.136 | 5.198 | 8.251 | 4.553 | 4.162 | 94.458 |
| Example 4 | 65.745 | 8.452 | 5.213 | 8.668 | 4.875 | 2.149 | 95.102 |
| Example 5 | 64.131 | 11.469 | 5.369 | 5.152 | 6.254 | 3.364 | 95.739 |
| Example 6 | 63.849 | 10.185 | 5.453 | 6.921 | 6.387 | 2.212 | 95.007 |
| Example 7 | 57.468 | 8.265 | 8.163 | 8.246 | 6.467 | 5.109 | 93.718 |
| Example 8 | 60.872 | 7.164 | 7.732 | 6.354 | 7.364 | 5.476 | 94.962 |
| Example 9 | 52.135 | 10.089 | 8.946 | 10.762 | 8.492 | 5.338 | 95.762 |
| Example 10 | 68.126 | 10.342 | 5.153 | 5.481 | 4.568 | 4.168 | 97.838 |
| Comparativ e Example 1 | 53.348 | 7.247 | 6.543 | 4.846 | 4.121 | 4.264 | 80.369 |
| Comparativ e Example 2 | 54.146 | 7.164 | 5.584 | 6.495 | 3.384 | 5.945 | 82.718 |
| Comparativ e Example 3 | 55.146 | 6.742 | 5.168 | 5.889 | 4.159 | 6.996 | 84.100 |
| Comparativ e Example 4 | 43.998 | 8.389 | 5.498 | 6.658 | 3.462 | 2.131 | 70.136 |
| Comparativ e Example 5 | 46.782 | 7.162 | 5.131 | 7.135 | 5.221 | 2.172 | 73.603 |
| Comparativ e Example 6 | 48.365 | 8.452 | 4.965 | 8.164 | 3.341 | 2.659 | 75.946 |
| Comparativ e Example 7 | 56.196 | 9.997 | 5.179 | 8.632 | 3.264 | 1.496 | 84.764 |
| Comparativ e Example 8 | 52.137 | 8.435 | 7.013 | 6.452 | 4.579 | 1.643 | 80.259 |
| Comparativ e Example 9 | 49.684 | 6.653 | 6.662 | 5.469 | 5.566 | 1.257 | 75.291 |
| Comparativ e Example 10 | 47.368 | 8.879 | 6.468 | 7.986 | 3.219 | 1.136 | 75.056 |

Table 3 Results of Weight-method Test in Examples 1-10 and Comparative Examples 1-10

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Example 1 | -0.0 765 | -0.11 81 | -0.3 340 | -0.3 376 | -0.4 201 | -0.4 875 | -0.5 096 | -0.5 955 | -0.6 773 | -0.7 472 | -0.8 279 | -0.9 095 | -1.1 355 | -1.2 078 |
| Example 2 | -0.0 376 | -0.0 607 | -0.2 264 | -0.2 793 | -0.3 320 | -0.4 013 | -0.4 142 | -0.4 361 | -0.4 747 | -0.5 942 | -0.6 664 | -0.7 409 | -0.8 215 | -0.9 948 |
| Example 3 | -0.0 916 | -0.1 394 | -0.2 168 | -0.3 412 | -0.4 025 | -0.4 551 | -0.4 839 | -0.5 664 | -0.5 818 | -0.6 346 | -0.7 238 | -0.8 674 | -0.9 412 | -1.0 941 |
| Example 4 | -0.0 747 | -0.1 336 | -0.3 158 | -0.4 265 | -0.5 312 | -0.5 894 | -0.6 143 | -0.7 152 | -0.7 998 | -0.8 125 | -0.8 759 | -0.9 414 | -0.9 945 | -1.1 304 |
| Example 5 | -0.0 666 | -0.0 842 | -0.1 214 | -0.2 345 | -0.3 058 | -0.3 377 | -0.3 954 | -0.4 316 | -0.4 913 | -0.5 258 | -0.7 135 | -0.8 468 | -0.9 245 | -1.0 753 |
| Example 6 | -0.0 415 | -0.0 964 | -0.1 324 | -0.2 625 | -0.3 142 | -0.3 869 | -0.4 312 | -0.5 613 | -0.6 462 | -0.7 325 | -0.8 443 | -0.8 917 | -0.9 614 | -1.11 55 |
| Example 7 | -0.0 843 | -0.1 212 | -0.2 468 | -0.3 213 | -0.3 942 | -0.4 168 | -0.5 152 | -0.5 262 | -0.5 501 | -0.5 914 | -0.6 484 | -0.7 312 | -0.8 128 | -0.9 613 |
| Example 8 | -0.0 685 | -0.0 914 | -0.2 215 | -0.3 131 | -0.3 224 | -0.3 876 | -0.4 142 | -0.4 665 | -0.5 124 | -0.5 867 | -0.6 014 | -0.6 973 | -0.7 463 | -0.9 246 |
| Example 9 | -0.0 762 | -0.1 346 | -0.1 989 | -0.3 245 | -0.4 613 | -0.4 987 | -0.6 646 | -0.6 942 | -0.7 171 | -0.7 845 | -0.8 362 | -0.9 012 | -0.9 977 | -1.1 493 |
| Example 10 | -0.0 668 | -0.8 614 | -0.1 358 | -0.2 121 | -0.3 649 | -0.4 351 | -0.6 133 | -0.7 492 | -0.8 372 | -0.9 615 | -0.9 884 | -1.1 001 | -1.3 215 | -1.4 862 |
| Comparative Example 1 | -0.0 456 | -0.0 690 | -0.1 772 | -0.2 003 | -0.2 307 | -0.2 639 | -0.2 903 | -0.2 997 | -0.3 419 | -0.4 006 | -0.4 349 | -0.4 718 | -0.4 903 | -0.5 902 |
| Comparative Example 2 | -0.0 432 | -0.0 717 | -0.1 213 | -0.1 894 | -0.2 021 | -0.2 139 | -0.2 422 | -0.2 968 | -0.3 154 | -0.3 939 | -0.4 261 | -0.5 352 | -0.5 954 | -0.6 742 |
| Comparative Example 3 | -0.0 514 | -0.0 863 | -0.1 846 | -0.2 189 | -0.2 426 | -0.2 913 | -0.3 145 | -0.3 432 | -0.4 202 | -0.4 658 | -0.4 867 | -0.5 051 | -0.5 203 | -0.7 316 |
| Comparative Example 4 | -0.0 323 | -0.0 541 | -0.0 812 | -0.11 33 | -0.1 496 | -0.1 927 | -0.2 032 | -0.2 376 | -0.2 814 | -0.2 997 | -0.3 030 | -0.31 18 | -0.3 914 | -0.4 321 |
| Comparative Example 5 | -0.0 584 | -0.0 852 | -0.2 482 | -0.2 601 | -0.2 919 | -0.3 039 | -0.3 232 | -0.3 695 | -0.3 735 | -0.3 991 | -0.4 077 | -0.4 329 | -0.4 464 | -0.4 511 |
| Comparative Example 6 | -0.0 413 | -0.0 732 | -0.1 062 | -0.1 346 | -0.1 539 | -0.1 732 | -0.2 020 | -0.2 463 | -0.2 935 | -0.3 345 | -0.3 664 | -0.4 092 | -0.4 512 | -0.5 043 |
| Comparative Example 7 | -0.0 610 | -0.0 938 | -0.11 15 | -0.2 324 | -0.2 698 | -0.3 135 | -0.3 754 | -0.4 114 | -0.4 839 | -0.5 122 | -0.6 045 | -0.6 213 | -0.6 517 | -0.6 732 |
| Comparative Example 8 | -0.0 664 | -0.1 010 | -0.1 336 | -0.2 048 | -0.2 724 | -0.3 369 | -0.3 846 | -0.4 221 | -0.5 425 | -0.6 483 | -0.6 759 | -0.6 996 | -0.7 003 | -0.7 012 |
| Comparative Example 9 | -0.0 379 | -0.0 642 | -0.0 94 | -0.1 032 | -0.1 989 | -0.2 192 | -0.2 864 | -0.3 006 | -0.3 195 | -0.3 769 | -0.4 141 | -0.4 265 | -0.4 542 | -0.4 664 |

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Comparative Example 10 | -0.0 404 | -0.0 712 | -0.0 986 | -0.1 214 | -0.1 394 | -0.1 958 | -0.2 324 | -0.2 758 | -0.3 009 | -0.3 412 | -0.3 958 | -0.4 225 | -0.4 641 | -0.4 815 |

It can be seen from the results of GC-MS test in Table 2 that the silica provided in Examples 1-10 had a sum of essences in a headspace gas of 90.906%-96.962% in GC-MS test, which was significantly higher than that in Comparative Examples 1-10, showing that the silica provided by the present disclosure had a weak capacity to adsorb an essence and better essence volatility, and had an advantage of reducing the cost of toothpaste.

It can be seen from the results of weight-method test in Table 3 that the silica provided in Examples 1-10 had an essence volatilizing weight of $\geq$0.9246 g (essence volatilizing weight proportion of $\geq$46.23%) at day 14, and had a weaker capacity to adsorb an essence, which was significantly superior to that in Comparative Examples 1-10. In the presence of the silica in Examples 1-10, the essence in the system was more volatile at room temperature, and the essence volatility was better.

**Example 11 Preparation of Silica with Low Specific Surface Area and Low Pore Volume by Post Treatment of Precipitation Method**

The silica prepared by the precipitation method in Comparative Example 7 was subjected to high-temperature calcination treatment as follows: a sample was prepared, the temperature of a muffle furnace was set to 800°C, and high-temperature calcination was carried out for 6 hours. After the high-temperature calcination, the internal pores of the silica collapsed, the pore volume was decreased, and the specific surface area was decreased accordingly.

**Example 12 Preparation of Silica with Low Specific Surface Area and Low Pore Volume by Post Treatment of Precipitation Method**

Compared with Example 11, this example only differs in that the temperature of a muffle furnace was set to 900°C, and high-temperature calcination was carried out for 3 hours. After the high-temperature calcination, the internal pores of the silica collapsed, the pore volume was decreased, and the specific surface area was decreased accordingly.

**Example 13 Preparation of Silica with Low Specific Surface Area and Low Pore Volume by Post Treatment of Precipitation Method**

Compared with Example 11, this example only differs in that the temperature of a muffle furnace was set to 1,000°C, and high-temperature calcination was carried out for 2 hours. After the high-temperature calcination, the internal pores of the silica collapsed, the pore volume was decreased, and the specific surface area was decreased accordingly.

**Comparative Example 11**

Compared with Example 11, this comparative example only differs in that the temperature of a muffle furnace was set to 500°C, and high-temperature calcination was carried out for 2 hours.

**Comparative Example 12**

Compared with Example 11, this comparative example only differs in that the temperature of a muffle furnace was set to 600°C, and high-temperature calcination was carried out for 4 hours.

**Comparative Example 13**

Compared with Example 11, this comparative example only differs in that the temperature of a muffle furnace was set to 800°C, and high-temperature calcination was carried out for 4 hours.

**Performance Test for Silica Prepared in Examples 11-13 and Comparative Examples 11-13**

The silica in Examples 11-13 of the present disclosure had a fluorine compatibility of more than 90% and a specific surface area of <2.79 m$^2$/g, which could satisfy the requirements of the European Union for non-nanomaterials, and the silica had a pore volume of 0.0006-0.02 cm$^3$/g and had excellent essence volatility. Moreover, the dispersive performance and cleaning performance were excellent.

However, in Comparative Examples 11-13, the specific surface area was >2.79 m$^2$/g, which could not satisfy the requirements of the European Union for non-nanomaterials; the pore volume was significantly higher than that in all the examples; the fluorine compatibility was also significantly lower than that in all the examples.

Table 4 Results of Performance Test for Silica in Examples 11-13 and Comparative Examples 11-13

| Item | Fluorine compatibility of powder (%) | BET (m²/g) | Pore volume cm³/g | Pore diameter nm | RDA | PCR | D50 μm | Apparent density g/ml | Oil absorption value g/100g | Copper loss mg |
|---|---|---|---|---|---|---|---|---|---|---|
| Example 11 | 90 | 0.0143 | 0.0000 8 | 22.4568 | 253.2 | 121.3 | 12.48 | 1.03 | 29 | 18.9 |
| Example 12 | 92 | 0.0091 | 0.0000 5 | 22.1446 | 258.6 | 123.2 | 12.53 | 0.99 | 32 | 18.7 |
| Example 13 | 91 | 0.0084 | 0.0000 4 | 18.8894 | 260.3 | 124.6 | 12.52 | 0.96 | 35 | 18.5 |
| Comparative Example 11 | 92 | 13.151 6 | 0.0814 | 24.7869 | 249.8 | 120.2 | 12.55 | 0.84 | 46 | 17.6 |
| Comparative Example 12 | 90 | 8.4416 | 0.0521 | 24.6954 | 250.2 | 120.1 | 12.51 | 0.86 | 42 | 17.9 |
| Comparative Example 13 | 91 | 4.9704 | 0.0303 | 24.3842 | 251.3 | 121.2 | 12.53 | 0.89 | 39 | 18.1 |

Table 5 Results of GC-MS Test in Examples 11-13 and Comparative Examples 11-13

| Item | Menthol (%) | Menthone (%) | Limonene (%) | Methyl salicylate (%) | Carvone (%) | Anethole (%) | Sum (%) |
|---|---|---|---|---|---|---|---|
| Example 11 | 68.141 | 10.125 | 3.368 | 5.731 | 6.142 | 4.147 | 97.654 |
| Example 12 | 67.342 | 10.464 | 4.132 | 4.464 | 5.261 | 5.184 | 96.847 |
| Example 13 | 65.987 | 10.071 | 4.047 | 5.132 | 5.554 | 5.484 | 96.275 |
| Comparativ e Example 11 | 56.132 | 9.947 | 6.246 | 6.189 | 4.142 | 1.447 | 84.103 |
| Comparativ e Example 12 | 57.894 | 8.732 | 7.701 | 5.131 | 5.445 | 2.149 | 87.052 |
| Comparativ e Example 13 | 57.421 | 10.129 | 7.732 | 5.124 | 4.478 | 2.649 | 87.533 |

Table 6 Results of Weight-method Test in Examples 11-13 and Comparative Examples 11-13

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Example 11 | -0.0 933 | -0.1 221 | -0.3 168 | -0.5 142 | -0.6 311 | -0.8 135 | -0.8 998 | -0.9 031 | -0.9 746 | -1.1 146 | -1.1 358 | -1.2 467 | -1.3 031 | -1.4 416 |
| Example 12 | -0.0 978 | -0.1 301 | -0.3 946 | -0.4 136 | -0.5 133 | -0.6 036 | -0.7 978 | -0.8 864 | -0.8 989 | -0.9 316 | -0.9 596 | -1.0 312 | -1.1 323 | -1.2 512 |
| Example 13 | -0.0 998 | -0.1 442 | -0.2 745 | -0.3 642 | -0.5 225 | -0.6 745 | -0.7 817 | -0.8 216 | -0.8 946 | -0.9 341 | -0.9 978 | -1.1 013 | -1.1 819 | -1.2 121 |
| Comparative Example 11 | -0.0 514 | -0.0 773 | -0.0 969 | -0.1 101 | -0.1 016 | -0.2 020 | -0.2 131 | -0.4 662 | -0.4 867 | -0.5 113 | -0.5 394 | -0.5 618 | -0.5 925 | -0.6 042 |
| Comparative Example 12 | -0.0 631 | -0.0 841 | -0.1 997 | -0.3 024 | -0.3 876 | -0.4 288 | -0.4 867 | -0.5 213 | -0.5 745 | -0.6 079 | -0.7 164 | -0.7 335 | -0.7 441 | -0.7 458 |
| Comparative Example 13 | -0.0 432 | -0.0 713 | -0.0 945 | -0.1 313 | -0.3 362 | -0.4 017 | -0.4 379 | -0.4 952 | -0.5 144 | -0.5 798 | -0.6 138 | -0.6 974 | -0.7 458 | -0.7 712 |

It can be seen from the results of GC-MS test in Table 5 that the silica provided in Examples 11-13 had a sum of essences in a headspace gas of 96.275%-97.654% in GC-MS test, which was significantly higher than that in Comparative Examples 11-13, showing that the silica provided by the present disclosure had a weak capacity to adsorb an essence and better essence volatility, and had an advantage of reducing the cost of toothpaste.

It can be seen from the results of weight-method test in Table 6 that the silica provided in Examples 11-13 had a weaker capacity to adsorb an essence, and the essence was more volatile at a room temperature, with an essence volatilizing weight of 1.2121-1.4416 g (essence volatilizing weight proportion of 60.605%-72.08%). The silica provided in Examples 11-13 had better essence volatility.

**Example 14 Preparation of Silica with Low Specific Surface Area and Low Pore Volume by Two-step Method Reaction**

1) 6 m$^3$ of a sodium sulfate solution with a mass percent concentration of 10% and 0.6 m$^3$ of a 1.5 mol/L sodium silicate solution were added to a reaction vessel, and heated up to 90°C-95°C;
2) stirring was carried out at a rotation speed of 400 revolutions per minute, a 1.5 mol/L sulfuric acid solution was added to the reaction vessel at a flow rate of 5 m$^3$/h, and the pH was adjusted to 7.0-7.5;
3) the sodium silicate solution and the sulfuric acid solution were slowly added simultaneously (acid and base were dropwise added simultaneously), with an addition speed of the sodium silicate solution being 5 m$^3$/h; and the pH of the solution was maintained at 7.0-7.5 by adjusting the flow rate of the sulfuric acid solution,
wherein silica particles growing under the condition of the process pH being partial neutral had poorer activity so that collision between particles could be reduced, the acid and base neutralization rate in a reaction system was higher, crystal grains grew rapidly, and since the end of a sol bond had high activity and lower aggregation density, active groups were easy to be close to each other, thereby promoting the growth of the bond, forming a linear chain structure which facilitated the formation of dense particles, and reduced the specific surface area of the particles;
4) the addition of the sodium silicate solution was stopped when the sodium silicate solution was added to 30 m$^3$ (at this time, the reaction time was about 21,600 seconds); and the sulfuric acid solution was added continuously until the pH of the solution was 4.5, and the addition of the sulfuric acid solution was stopped;
5) stirring continued, heat preservation for aging was carried out for 30 minutes to prepare a silica slurry; and
6) the silica slurry was filtered with a diaphragm, and the sodium sulfate solution was recovered; and then washing, spray-drying, and jet mill crushing were carried out to prepare silica with a low specific surface area and a low pore volume.

**Example 15 Preparation of Silica with Low Specific Surface Area and Low Pore Volume by Two-step Method Reaction**

Compared with Example 14, this example only differs in that the reaction rotation speed was adjusted to 600 revolutions per minute.

**Comparative Example 14**

Compared with Example 14, this comparative example only differs in that the process pH was adjusted to 6.0-6.5.

**Comparative Example 15**

Compared with Example 14, this comparative example only differs in that the addition speed (flow rate) of the sodium silicate solution in step 3) was 40 m$^3$/h.

**Performance Test for Silica Prepared in Examples 14-15 and Comparative Examples 14-15**

The silica in Examples 14-15 had a fluorine compatibility of more than 90% and a specific surface area of <2.79 m$^2$/g, which could satisfy the requirements of the European Union for non-nanomaterials, and the silica had a pore volume of 0.0008-0.0009 cm$^3$/g and had excellent essence volatility. Moreover, the dispersive performance and cleaning performance were excellent.

However, the silica in Comparative Examples 14-15 had a specific surface area of >2.79 m$^2$/g, which could not satisfy the requirements of the European Union for non-nanomaterials; the pore volume was significantly higher than that in all the examples; the fluorine compatibility was also significantly lower than that in all the examples.

Table 7 Results of Performance Test for Silica in Examples 14-15 and Comparative Examples 14-15

| Item | Fluorine compatibility of powder (%) | BET (m²/g) | Pore volume cm³/g | Pore diameter nm | RDA | PCR | D50 μm | Apparent density g/ml | Oil absorption value g/100g | Copper loss mg |
|---|---|---|---|---|---|---|---|---|---|---|
| Example 14 | 93 | 0.1231 | 0.0008 | 25.9853 | 243.6 | 118.7 | 10.14 | 0.93 | 36.8 | 17.3 |
| Example 15 | 92 | 0.1421 | 0.0009 | 25.3369 | 248.9 | 119.4 | 10.69 | 0.97 | 32.5 | 17.8 |
| Comparative Example 14 | 88 | 3.1358 | 0.0165 | 20.0142 | 232.1 | 115.6 | 12.58 | 0.89 | 43.6 | 16.8 |
| Comparative Example 15 | 89 | 5.5705 | 0.0359 | 25.7787 | 229.4 | 114.3 | 11.21 | 0.88 | 42.1 | 16.4 |

Table 8 Results of GC-MS Test in Examples 14-15 and Comparative Examples 14-15

| Item | Menthol (%) | Menthone (%) | Limonene (%) | Methyl salicylate (%) | Carvone (%) | Anethole (%) | Sum (%) |
|---|---|---|---|---|---|---|---|
| Example 14 | 69.732 | 11.248 | 2.264 | 4.641 | 5.896 | 4.448 | 98.229 |
| Example 15 | 69.464 | 11.145 | 3.148 | 5.627 | 3.249 | 5.436 | 98.069 |
| Comparativ e Example 14 | 56.594 | 8.008 | 6.132 | 4.554 | 3.249 | 2.313 | 80.850 |
| Comparativ e Example 15 | 57.662 | 9.478 | 5.949 | 4.681 | 4.648 | 3.012 | 85.430 |

Table 9 Results of Weight-method Test in Examples 14-15 and Comparative Examples 14-15

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Example 14 | -0.0 843 | -0.0 996 | -0.1 224 | -0.2 867 | -0.4 164 | -0.5 638 | -0.8 312 | -0.9 746 | -1.1 318 | -1.2 446 | -1.3 553 | -1.4 762 | -1.5 174 | -1.5 212 |
| Example 15 | -0.0 762 | -0.1 013 | -0.1 941 | -0.3 062 | -0.4 438 | -0.6 162 | -0.8 994 | -0.9 475 | -1.0 321 | -1.3 838 | -1.4 211 | -1.4 934 | -1.5 101 | -1.5 358 |
| Comparative Example 14 | -0.0 533 | -0.7 894 | -0.9 296 | -0.1 203 | -0.1 735 | -0.2 396 | -0.2 735 | -0.3 464 | -0.3 987 | -0.4 612 | -0.5 012 | -0.5 968 | -0.6 384 | -0.6 664 |
| Comparative Example 15 | -0.0 497 | -0.0 665 | -0.0 819 | -0.1 013 | -0.2 167 | -0.2 945 | -0.3 163 | -0.4 358 | -0.4 938 | -0.5 216 | -0.6 101 | -0.6 974 | -0.7 171 | -0.7 916 |

It can be seen from the results of GC-MS test in Table 8 that the silica provided in Examples 14-15 had a sum of essences in a headspace gas of 98.069%-98.229% in GC-MS test, which was significantly higher than that in Comparative Examples 14-15, showing that the silica provided by the present disclosure had a weak capacity to adsorb an essence and better essence volatility, and had an advantage of reducing the cost of toothpaste.

It can be seen from the results of weight-method test in Table 9 that the silica provided in Examples 14-15 had a weaker capacity to adsorb an essence, and the essence was more volatile at a room temperature, with an essence volatilizing weight of 1.5212-1.5358 g (essence volatilizing weight proportion of 76.08%-76.79%). The silica provided in Examples 14-15 had better essence volatility.

**Example 16 Preparation of Silica with Low Specific Surface Area and Low Pore Volume by Post Treatment of Two-step Method**

The silica prepared by two-step method in Comparative Example 15 was subjected to high-temperature calcination treatment as follows: a sample was prepared, the temperature of a muffle furnace was set to 800°C, and high-temperature calcination was carried out for 3 hours. After the high-temperature calcination, the internal pores of the silica collapsed, the pore volume was decreased, and the specific surface area was decreased accordingly.

**Example 17 Preparation of Silica with Low Specific Surface Area and Low Pore Volume by Post Treatment of Two-step Method**

The silica prepared by two-step method in Comparative Example 15 was subjected to high-temperature calcination treatment as follows: a sample was prepared, the temperature of a muffle furnace was set to 800°C, and high-temperature calcination was carried out for 2 hours. After the high-temperature calcination, the internal pores of the silica collapsed, the pore volume was decreased, and the specific surface area was decreased accordingly.

**Comparative Example 16 Preparation of Silica with Low Specific Surface Area and Low Pore Volume by Post Treatment of Two-step Method**

The silica prepared by two-step method in Comparative Example 15 was subjected to high-temperature calcination treatment as follows: a sample was prepared, the temperature of a muffle furnace was set to 500°C, and high-temperature calcination was carried out for 3 hours.

**Comparative Example 17 Preparation of Silica with Low Specific Surface Area and Low Pore Volume by Post Treatment of Two-step Method**

The silica prepared by two-step method in Comparative Example 15 was subjected to high-temperature calcination treatment as follows: a sample was prepared, the temperature of a muffle furnace was set to 500°C, and high-temperature calcination was carried out for 5 hours.

The silica in Examples 16-17 had a fluorine compatibility of more than 90% and a specific surface area of <2.79 $m^2$/g, which could satisfy the requirements of the European Union for non-nanomaterials, and the silica had a pore volume of 0.0005-0.0006 $cm^3$/g and had excellent essence volatility. Moreover, the dispersive performance and cleaning performance were excellent.

However, the silica in Comparative Examples 16-17 had a specific surface area of >2.79 $m^2$/g, which could not satisfy the requirements of the European Union for non-nanomaterials; the pore volume was significantly higher than that in all the examples; the fluorine compatibility was also significantly lower than that in all the examples.

Table 10 Results of Performance Test for Silica in Examples 16-17 and Comparative Examples 16-17

| Item | Fluorine compatibility of powder (%) | BET ($m^2$/g) | Pore volume $cm^3$/g | Pore diameter nm | RDA | PCR | D50 μm | Apparent density g/ml | Oil absorption value g/100g | Copper loss mg |
|---|---|---|---|---|---|---|---|---|---|---|
| Example 16 | 94 | 0.0789 | 0.0005 | 25.3469 | 245.9 | 121.3 | 11.23 | 0.94 | 35.4 | 17.2 |
| Example 17 | 92 | 0.0943 | 0.0006 | 25.4264 | 244.3 | 121.1 | 11.20 | 0.92 | 37.3 | 17.1 |

(continued)

| Item | Fluorine compatibility of powder (%) | BET (m2/g) | Pore volume cm3/g | Pore diameter nm | RDA | PCR | D50 μm | Apparent density g/ml | Oil absorption value g/100g | Copper loss mg |
|---|---|---|---|---|---|---|---|---|---|---|
| Comparative Example 16 | 89 | 4.9266 | 0.0315 | 25.5752 | 236.5 | 123.5 | 11.21 | 0.89 | 42.3 | 16.8 |
| Comparative Example 17 | 88 | 4.8237 | 0.0308 | 25.5384 | 242.4 | 124.2 | 11.22 | 0.91 | 40.1 | 16.6 |

Table 11 Results of GC-MS Test in Examples 16-17 and Comparative Examples 16-17

| Item | Menthol (%) | Menthone (%) | Limonene (%) | Methyl salicylate (%) | Carvone (%) | Anethole (%) | Sum (%) |
|---|---|---|---|---|---|---|---|
| Example 16 | 69.942 | 11.038 | 1.649 | 4.436 | 5.969 | 5.552 | 98.586 |
| Example 17 | 70.468 | 11.674 | 2.132 | 4.348 | 5.012 | 4.449 | 98.083 |
| Comparativ e Example 16 | 56.369 | 10.204 | 3.978 | 5.255 | 5.168 | 4.349 | 85.323 |
| Comparativ e Example 17 | 57.318 | 10.003 | 3.732 | 5.649 | 5.138 | 3.274 | 85.114 |

Table 12 Results of Weight-method Test in Examples 16-17 and Comparative Examples 16-17

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Example 16 | -0.0 901 | -0.1 194 | -0.2 654 | -0.4 104 | -0.4 658 | -0.5 775 | -0.7 654 | -0.8 745 | -0.9 192 | -1.0 002 | -1.1 121 | -1.2 648 | -1.3 614 | -1.5 434 |
| Example 17 | -0.0 931 | -0.1 350 | -0.2 984 | -0.3 687 | -0.4 791 | -0.5 858 | -0.7 071 | -0.8 132 | -0.8 976 | -0.9 135 | -1.1 358 | -1.2 664 | -1.4 769 | -1.5 202 |
| Comparative Example 16 | -0.0 639 | -0.0 811 | -0.1 042 | -0.2 158 | -0.3 162 | -0.3 745 | -0.4 162 | -0.5 735 | -0.6 064 | -0.6 369 | -0.6 512 | -0.6 774 | -0.6 963 | -0.7 552 |
| Comparative Example 17 | -0.0 594 | -0.0 914 | -0.1 113 | -0.1 958 | -0.2 142 | -0.3 049 | -0.3 658 | -0.4 132 | -0.4 994 | -0.5 168 | -0.6 431 | -0.7 003 | -0.7 018 | -0.7 077 |

It can be seen from the results of GC-MS test in Table 11 that the silica provided in Examples 16-17 had a sum of essences in a headspace gas of 98.083%-98.586% in GC-MS test, which was significantly higher than that in Comparative Examples 16-17, showing that the silica provided by the present disclosure had a weak capacity to adsorb an essence and better essence volatility, and had an advantage of reducing the cost of toothpaste.

It can be seen from the results of weight-method test in Table 12 that the silica provided in Examples 16-17 had a weaker capacity to adsorb an essence, and the essence was more volatile at a room temperature, with an essence volatilizing weight of 1.5202-1.5434 g (essence volatilizing weight proportion of 76.01%-77.17%). The silica provided in Examples 16-17 had better essence volatility.

**Comparative Example 18**

Compared with Example 14, this comparative example only differs in that the process pH was adjusted to 4.0-4.5.

**Comparative Example 19**

Compared with Example 1, this comparative example only differs in that the reaction temperature in step 3) was maintained at 40°C.

**Comparative Example 20**

Compared with Example 1, this comparative example only differs in that the sodium sulfate solution had a mass percent concentration of 1%.

Table 13 Results of Performance Test for Silica in Comparative Examples 18-20

| Item | Fluorine compatibility of powder (%) | BET (m²/g) | Pore volume cm³/g | Pore diameter nm | RDA | PCR | D50 μm | Apparent density g/ml | Oil absorption value g/100g | Copper loss mg |
|---|---|---|---|---|---|---|---|---|---|---|
| Comparative Example 18 | 87 | 2.1928 | 0.0022 | 4.0131 | 244.8 | 118.4 | 11.12 | 0.92 | 33.6 | 17.7 |
| Comparative Example 19 | 83 | 37.504 6 | 0.0979 | 10.4425 | 193.1 | 106.8 | 14.36 | 0.79 | 79 | 13.8 |
| Comparative Example 20 | 85 | 40.860 6 | 0.1171 | 11.4618 | 189.6 | 101.2 | 12.32 | 0.71 | 87 | 12.2 |

Table 14 Results of GC-MS Test in Comparative Examples 18-20

| Item | Menthol (%) | Menthone (%) | Limonene (%) | Methyl salicylate (%) | Carvone (%) | Anethole (%) | Sum (%) |
|---|---|---|---|---|---|---|---|
| Comparativ e Example 18 | 38.315 | 6.134 | 3.246 | 4.462 | 3.158 | 4.1414 | 59.456 |
| Comparativ e Example 19 | 43.348 | 5.137 | 6.543 | 3.846 | 3.121 | 2.264 | 64.259 |
| Comparativ e Example 20 | 44.771 | 4.361 | 7.102 | 3.341 | 4.466 | 4.782 | 68.823 |

Table 15 Results of Weight-method Test in Comparative Examples 18-20

| | 1d (g) | 2d (g) | 3d (g) | 4d (g) | 5d (g) | 6d (g) | 7d (g) | 8d (g) | 9d (g) | 10d (g) | 11d (g) | 12d (g) | 13d (g) | 14d (g) |
|---|---|---|---|---|---|---|---|---|---|---|---|---|---|---|
| Comparative Example 18 | -0.0 699 | -0.0 829 | -0.0 9564 | -0.09 745 | -0.09 812 | -0.09 906 | -0.1 082 | -0.1 464 | -0.1 859 | -0.2 016 | -0.2 281 | -0.2 309 | -0.2 425 | -0.2 564 |
| Comparative Example 19 | -0.1 065 | -0.1 458 | -0.1 771 | -0.19 56 | -0.19 85 | -0.20 88 | -0.2 455 | -0.2 619 | -0.2 626 | -0.2 776 | -0.2 848 | -0.2 907 | -0.3 012 | -0.3 126 |
| Comparative Example 20 | -0.0 813 | -0.0 936 | -0.1 245 | -0.13 65 | -0.16 82 | -0.18 94 | -0.1 913 | -0.2 112 | -0.2 365 | -0.2 492 | -0.2 772 | -0.2 843 | -0.2 934 | -0.3 011 |

It can be seen from the results of test for performance of silica in Table 13 and the results of GC-MS test in Table 14 that the silica is a porous material and can adsorb an essence, and the silica with a smaller pore diameter has a better capacity to adsorb an essence, so that the essence is bound in the pore diameter of the silica and cannot be easily volatilized; for the silica with a larger pore diameter, the essence can be volatilized better. Therefore, in Comparative Example 18, the pore diameter thereof was relatively small, so that the essence volatility was poorer, and it was difficult to achieve the technical effect of the present disclosure.

It can be seen from the results of weight-method test in Table 15 that when the silica had a smaller pore diameter and a larger specific surface area, the silica had a stronger capacity to adsorb an essence, the essence was less volatile at a room temperature, and the essence volatility was poorer.

The above examples are preferred embodiments of the present disclosure, but the embodiments of the present disclosure are not limited by the above examples. Any other alteration, modification, substitution, combination and simplification made without departing from the spirit and principle of the present disclosure shall all be equivalent substitution, and shall all fall within the scope of protection of the present disclosure.

**Claims**

1. An abrasive-type silica for toothpaste with a low specific surface area and a low pore volume, **characterized in that**, the abrasive-type silica has:

   (1) a specific surface area of 0.01-2.79 $m^2$/g;
   (2) a pore volume of 0.00004-0.3 $cm^3$/g;
   (3) a pore diameter of 5-26 nm; and
   (4) a median particle diameter D50 of 1-25 $\mu$m.

2. The abrasive-type silica for toothpaste with a low specific surface area and a low pore volume according to claim 1, wherein

   (1) the specific surface area is: 0.01-2.79 $m^2$/g;
   (2) the pore volume is: 0.00004-0.1 $cm^3$/g;
   (3) the pore diameter is: 15-26 nm; and
   (4) the median particle diameter D50 is: 1-20 $\mu$m.

3. The abrasive-type silica for toothpaste with a low specific surface area and a low pore volume according to claim 2, wherein

   (1) the specific surface area is: 0.1-2.5 $m^2$/g;
   (2) the pore volume is: 0.00004-0.02 $cm^3$/g;
   (3) the pore diameter is: 15-26 nm; and
   (4) the median particle diameter D50 is: 1-16 $\mu$m.

4. The abrasive-type silica for toothpaste with a low specific surface area and a low pore volume according to any one of claims 1-3, wherein the abrasive-type silica has:

   (5) a fluorine compatibility of 75%-99%;
   (6) a copper sheet wear value of 6-25 mg per 10,000 revolutions;
   (7) an RDA value of 180-360;
   (8) a PCR value of 80-160;
   (9) an apparent density of 0.02-1.5 g/ml; and
   (10) an oil absorption value of 10-125 g/100g.

5. The abrasive-type silica for toothpaste with a low specific surface area and a low pore volume according to claim 4, wherein

   (5) the fluorine compatibility is: 80%-99%;
   (6) the copper sheet wear value is: 10-20 mg per 10,000 revolutions;
   (7) the RDA value is: 180-260;
   (8) the PCR value is: 100-160;

(9) the apparent density is: 0.3-1.5 g/ml; and

(10) the oil absorption value is: 10-60 g/100g.

6. The abrasive-type silica for toothpaste with a low specific surface area and a low pore volume according to claim 5, wherein

(5) the fluorine compatibility is: 90%-99%;

(6) the copper sheet wear value is: 10-20 mg per 10,000 revolutions;

(7) the RDA value is: 200-250;

(8) the PCR value is: 100-160;

(9) the apparent density is: 0.5-1.0 g/ml; and

(10) the oil absorption value is: 10-60 g/100g.

7. The abrasive-type silica for toothpaste with a low specific surface area and a low pore volume according to any one of claims 1 - 6, wherein the abrasive-type silica has an essence volatility measured as follows:

GC-MS is used for test, and the essence volatility is ≥90%; and a test method comprises: adding an essence into the abrasive-type silica or a toothpaste comprising the abrasive-type silica, and using the GC-MS for test to measure a proportion of the essence in a headspace gas, i.e., the essence volatility;

or a weight method is used for test, and the essence volatility for 14 days is ≥40%; and a test method comprises: adding the essence into the abrasive-type silica or the toothpaste comprising the abrasive-type silica, and testing a change in total weight after 14 days, wherein the change in total weight tested is the essence volatility.

8. Use of the abrasive-type silica for toothpaste with a low specific surface area and a low pore volume according to any one of claims 1-7 in preparation of an oral abrasive, a cleaning aid, or toothpaste.

9. An oral composition, comprising the abrasive-type silica for toothpaste with a low specific surface area and a low pore volume according to any one of claims 1-7, and an oral acceptable carrier.

10. The oral composition according to claim 9, wherein a content of the abrasive-type silica is 0.5%-90.0% by mass, and preferably, the content of the abrasive-type silica is 1.0%-80.0% by mass.

11. The oral composition according to claim 9 or 10, wherein the abrasive-type silica is present as a sole abrasive in the oral composition, or as an abrasive and/or a cleaning aid.

12. The oral composition according to any one of claims 9-11, wherein the oral composition is toothpaste.

13. A preparation method for the abrasive-type silica for toothpaste with a low specific surface area and a low pore volume according to any one of claims 1-7, comprising the following steps:

1) adding a sodium sulfate solution into a reaction vessel;

2) adding a sodium silicate solution and stirring well until a pH of a reaction mixture is 7-14;

3) heating up until a temperature of the reaction mixture is 60°C-97°C; and maintaining a reaction temperature, stirring at a rotation speed of 200-600 revolutions per minute, slowly adding the sodium silicate solution and a sulfuric acid solution simultaneously, with a flow rate of the sodium silicate solution being 5-30 m³/h, and maintaining a pH within a range of 7-10 by adjusting a flow rate of the sulfuric acid solution;

4) continuously adding the sodium silicate solution until a volume ratio of the sodium silicate solution to the sodium sulfate solution in step 1) is (1.5-2): 1; and continuously adding the sulfuric acid solution until a pH of a solution reaches 4-7, and stopping addition of the sulfuric acid solution;

5) continuing to stir, carrying out heat preservation for aging for 10-60 minutes to prepare a silica slurry; and

6) subjecting the silica slurry to filtering, washing, drying, and crushing to obtain a finished product,

wherein the sodium sulfate solution has a mass percent concentration of 6%-20%;

the sodium silicate solution has a concentration of 1-2.5 mol/L; and

the sulfuric acid solution has a concentration of 1-2 mol/L.

14. A preparation method for the abrasive-type silica for toothpaste with a low specific surface area and a low pore volume according to any one of claims 1-7, comprising the following steps:

1) adding a sodium sulfate solution and a sodium silicate solution into a reaction vessel, and heating up to 90°C-95°C;

2) stirring at a rotation speed of 200-600 revolutions per minute, adding a sulfuric acid solution into the reaction vessel, and adjusting a pH to 7.0-7.5;

3) slowly adding the sodium silicate solution and the sulfuric acid solution simultaneously, with an addition speed of the sodium silicate solution being 5-20 $m^3$/h; and maintaining a pH of a reaction mixture at 7.0-7.5 by adjusting a flow rate of the sulfuric acid solution;

4) continuously adding the sodium silicate solution until a volume ratio of the sodium silicate solution to the sodium sulfate solution in step 1) is (3-8): 1; and continuously adding the sulfuric acid solution until a pH of a solution is 4-7, and stopping addition of the sulfuric acid solution;

5) continuing to stir, carrying out heat preservation for aging for 30 minutes to prepare a silica slurry; and

6) subjecting the silica slurry to filtering, washing, drying, and crushing to prepare silica with a low specific surface area and a low pore volume,

  wherein the sodium sulfate solution has a mass percent concentration of 6%-20%;
  the sodium silicate solution has a concentration of 1-2.5 mol/L; and
  the sulfuric acid solution has a concentration of 1-2 mol/L.

15. An abrasive-type silica obtained by the preparation method according to claim 13 or 14, having a specific surface area of 0.01-2.79 $m^2$/g and a pore volume of 0.0006-0.3 $cm^3$/g.

| 5 µm | EHT = 2.00 kV | Signal A = SE2 | Date :12 Mar 2024 | ZEISS |
| | WD = 6.2 mm | Mag = 2.00 K X | Time :11:07:50 | |

FIG. 1

| 3 µm | EHT = 2.00 kV | Signal A = SE2 | Date :12 Mar 2024 | ZEISS |
| | WD = 6.1 mm | Mag = 3.00 K X | Time :11:10:04 | |

FIG. 2

FIG. 3

FIG. 4

Europäisches
Patentamt
European
Patent Office
Office européen
des brevets

# EUROPEAN SEARCH REPORT

**Application Number**

EP 25 16 0454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/009168 A1 (MADHU SILICA PVT LTD [IN]) 11 January 2024 (2024-01-11) * claims 1,9-11; examples 1, 4, 6; tables 1,4,6,8,9 * * the whole document * | 1-12,15 | INV. C01B33/193 A61K8/02 A61K8/25 A61Q11/00 |
| X | US 2024/051835 A1 (MOTL NATHAN [US] ET AL) 15 February 2024 (2024-02-15) * claim 3; examples 1A-1D,1J; tables 1-3,5,6 * * the whole document * | 1-12,15 | |
| X | CN 113 023 736 B (GUANGZHOU FEIXUE MATERIAL TECH CO LTD ET AL.) 19 November 2021 (2021-11-19) * Comparative examples 1-6; claim 1; example 1 * * the whole document * | 13 | |
| A | WO 2020/098272 A1 (GUANGZHOU FEIXUE MATERIAL TECH LTD [CN]) 22 May 2020 (2020-05-22) * claims 1-8; example 1 * * the whole document * | 14 | **TECHNICAL FIELDS SEARCHED (IPC)** C01B A61Q A61K |
| A | CN 111 232 995 A (GUANGZHOU FEIXUE MATERIAL TECH CO LTD ET AL.) 5 June 2020 (2020-06-05) * claims 1-5; examples 1-5 * * the whole document * | 14 | |

The present search report has been drawn up for all claims

| Place of search | Date of completion of the search | Examiner |
|---|---|---|
| The Hague | 5 March 2026 | Follens, Lana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

EP 4 768 437 A1

Europäisches
Patentamt
European
Patent Office
Office européen
des brevets

**Application Number**

EP 25 16 0454

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

# LACK OF UNITY OF INVENTION
# SHEET B

**Application Number**

EP 25 16 0454

The Search Division considers that the present European patent application does not comply with the requirements of unity of invention and relates to several inventions or groups of inventions, namely:

    1. claims: 1-12, 15

        Silica and use thereof
                ---

    2. claim: 13

        precipitation method to make silica
                ---

    3. claim: 14

        two-step method to make silica
                ---

## ANNEX TO THE EUROPEAN SEARCH REPORT
## ON EUROPEAN PATENT APPLICATION NO.

EP 25 16 0454

This annex lists the patent family members relating to the patent documents cited in  the above-mentioned European search report.
The members are as contained in the European Patent Office EDP file on
The European Patent Office is in no way liable for these particulars which are merely given for the purpose of information.

05-03-2026

| Patent document cited in search report | | Publication date | Patent family member(s) | | Publication date |
|---|---|---|---|---|---|
| WO 2024009168 | A1 | 11-01-2024 | NONE | | |
| US 2024051835 | A1 | 15-02-2024 | BR | 112023016180 A2 | 12-12-2023 |
| | | | CN | 116887802 A | 13-10-2023 |
| | | | EP | 4291153 A1 | 20-12-2023 |
| | | | JP | 2024508721 A | 28-02-2024 |
| | | | KR | 20230138530 A | 05-10-2023 |
| | | | TW | 202302065 A | 16-01-2023 |
| | | | US | 2024051835 A1 | 15-02-2024 |
| | | | WO | 2022171507 A1 | 18-08-2022 |
| CN 113023736 | B | 19-11-2021 | NONE | | |
| WO 2020098272 | A1 | 22-05-2020 | CN | 109264728 A | 25-01-2019 |
| | | | WO | 2020098272 A1 | 22-05-2020 |
| CN 111232995 | A | 05-06-2020 | NONE | | |

For more details about this annex : see Official Journal of the European Patent Office, No. 12/82

**EP 4 768 437 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201810273705
- US 12145852 B2

- GB 435762023 T

**Non-patent literature cited in the description**

- Commission Recommendation 2011/696/EU. 18 October 2011, 38